# EUROPEAN PATENT SPECIFICATION

(11) **EP 2 282 678 B1**
(45) Date of publication and mention of the grant of the patent: **27.03.2013**
(21) Application number: 09758638.2
(22) Date of filing: 05.06.2009
(51) Int. Cl.: A61B 17/04

(54) **A REMOTE CONTROL MECHANISM FOR ATRAUMATIC SURGICAL NEEDLE**
FERNSTEUERUNGSMECHANISMUS FÜR EINE ATRAUMATISCHE CHIRURGISCHE NADEL
MECANISME DE COMMANDE A DISTANCE POUR AIGUILLE CHIRURGICALE ATRAUMATIQUE

(30) Priority: 06.06.2008 TR 200804140
(43) Date of publication of application: 16.02.2011
(73) Proprietor: Aksakal, Orhan Seyfi, Ankara (TR)
(72) Inventor: Aksakal, Orhan Seyfi, Ankara (TR)
(74) Representative: Kangasmäki, Reijo Holger
(86) International application number: PCT/TR2009/000070
(87) International publication number: WO 2009/148415

(56) References cited:
- EP-A- 0 705 568
- WO-A-2005/018467
- WO-A-2007/135629
- US-A- 3 090 386
- US-A- 5 632 751
- US-A- 6 146 392

## Description

### Technical Field :

Invention will be facilitiated for the devices which are used for deep suturing or laparoscopic suturing where there should be a distance between the hand that gives the movement to the needle and the place to be sutured. It is developed for adaptation of the needle slot to the needles of different sizes to grip various sizes of needles and enabling the needle to pass through the tissue (flesh), and to be captured and doing all these by the same movement of the fingers of the hand holding the device.

There is no such mechanism used for the atraumatic surgical needles which are present in the market and currently in use.

The invention aims to bring solution to the following technical problems:
Invention control system will be used for fixing the atraumatic needle, upon which the suture is fixed, in its slot tightly; make the same slot to be adapted for different sizes of needles with a high level of consistency (to prevent the thin needles from tottering in the slot) and for allowing the needle to be passed through the tissue (flesh), and to be captured and to be released with a single hand movement of the surgeon for example; single push of the surgeon's thumb. These will speed the operation and will provide ease of use for the needle. In the current conditions, needle is not fixed in the slot, so it is tottered, especially for the thinner needles causing difficulty to find the target or it displays a risk for needle to fall accidently. Remote control mechanisms for a traumatic manipulation of surgical needles are disclosed in US 3 090 386 and in WO 2007 135 629. Besides, in the current conditions, the mechanism that provides movement to the needle and the mechanism that fixes the needle are seperately controlled and this fact causes distraction to the surgeon in the difficult operation circumstances. The invention will perform these same movements at the same time preventing the mentioned drawbacks being occurred.

### Explanation Of The Figures:

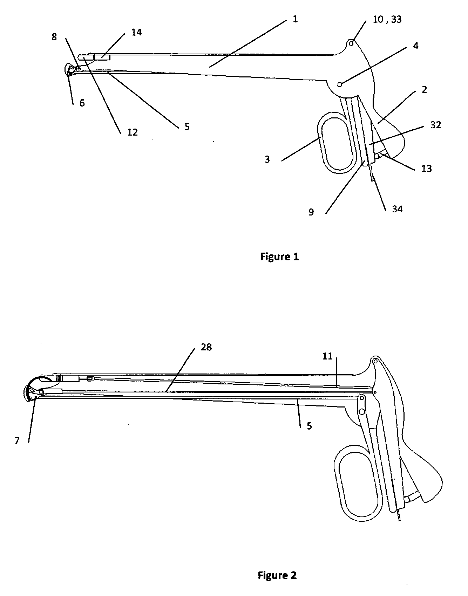

Figure 1 - Section view
Figure 2 - Section internal view

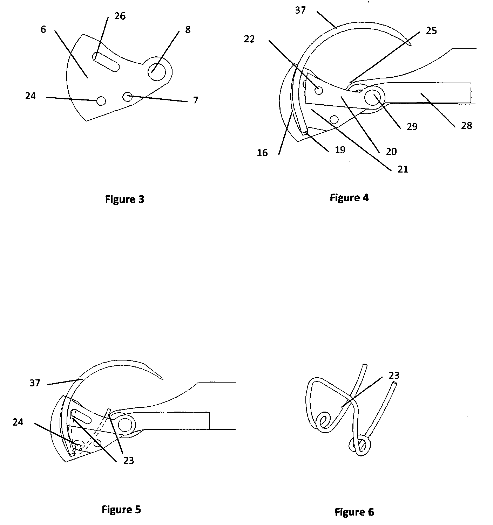

Figure 3 - Needle slot external side view
Figure 4 - Needle is in slot without fixed
Figure 5 - Needle is in slot and clamp is thrusting
Figure 6 - Spring oblique

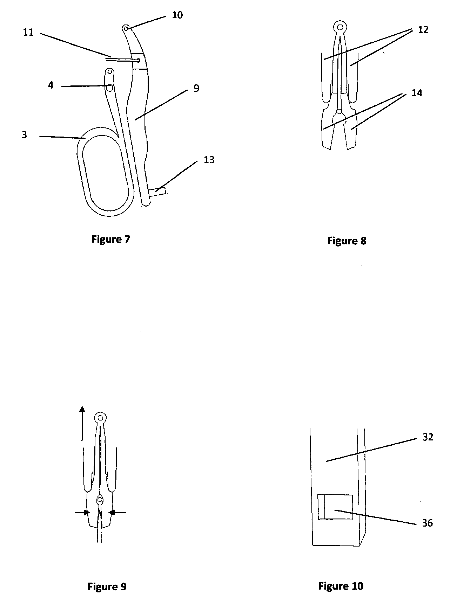

Figure 7 - Needle capture handle
Figure 8 - Needle capture grips are open
Figure 9 - Needle capture grips fixed the needle
Figure 10 - Needle capture handle detail - lock window

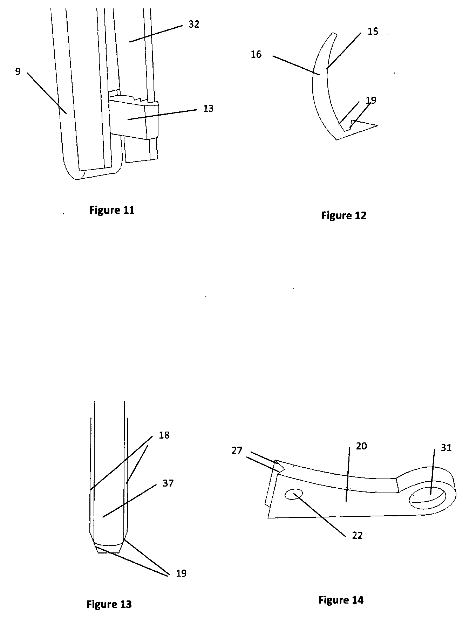

Figure 11 - The connection between needle capture and needle clamp handles
Figure 12 - The conical structure of the needle channel - side view
Figure 13 - The conical structure of the needle channel - front and rear
Figure 14 - Needle clamp tilted sides

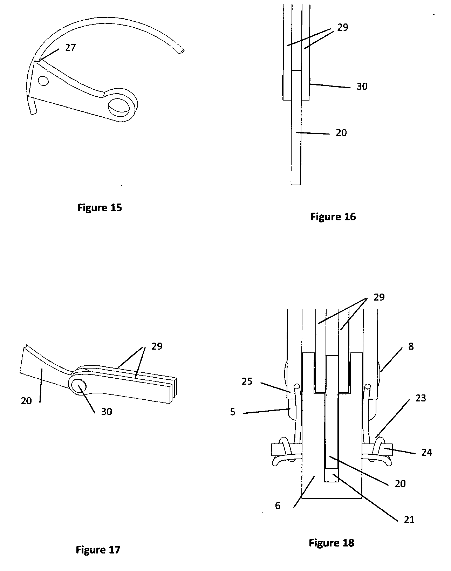

Figure 15 - The relation between the needle clamp and the needle
Figure 16 - The connection between the clamp and the control parts - top plan view
Figure 17 - The connection between the clamp and the control parts - oblique
Figure 18 - End parts connection - top plan view

### Part Numbers;

1- Body
2- A handle fixed to the body
3- Needle slot thrusting handle
4- Pivot point for the needle slot thrusting handle
5- Control rod for the needle slot
6- Needle slot
7- Hole for the needle slot control rod
8- Pivot point for the needle slot
9- Control handle for needle capture grip (jaws)
10- Pivot point of control handle for needle capture grip (jaws)
11- Control rod for needle capture grip
12- Needle capture grips
13- The lock mechanism of control handle for needle capture grip
14- Clamp side supports
15- Needle channel
16- Back side of needle channel
17- Front side of needle channel
18- Side walls for needle channel
19- Conical shaped needle channel bottom
20- Needle clamp
21- Needle clamp slot
22- Spring hole
23- Spring
24- Spring pivot point shaft
25- The side of body extension
26- Spring window (aperture)
27- Curved needle clamp sides
28- Needle channel opening wire
29- Clamp control parts
30- Clamp connection rod
31- Clamp connection hole
32- Needle channel opener handle
33- Needle channel opener handle pivot point
34- Release button for the needle channel
35- Needle channel opener handle spring
36- Hole of lock mechanism
37- Needle

### Explanation Of The Invention:

The invention comprising; a body (1), a handle fixed to the body (2), needle slot thrusting handle (3), pivot point for the needle slot thrusting handle (4), control rod for the needle slot (5), needle slot (6), needle slot control rod hole (7), needle slot pivot point (8), control handle for needle capture grip (9), pivot point of control handle for needle capture grip (10), control rod for needle capture grip (11), needle capture grips (12), the lock mechanism of control handle for needle capture grip (13), clamp side supports (14), needle channel (15), back side of needle channel (16), front side of needle channel (17), side walls for needle channel (18), conical shaped needle channel bottom (19), needle clamp (20), needle clamp slot (21), spring hole (22), spring (23), spring pivot point shaft (24), the side of body extension (25), spring window (26), curved needle clamp sides (27), needle channel opening wire (28), clamp control parts (29), clamp connection rod (30), clamp connection hole (31), needle channel opener handle (32), needle channel opener handle pivot point (33), release button for the needle channel (34), needle channel opener handle spring (35), hole of lock mechanism (36).

Body (1) connects the controls and the needle in remote control mechanism for atraumatic needle. Body can also be made circular and be passed through a laparoscopic trocar. The body length can be extended on demand so that suture control distance would be longer.

User, will command the tool by handle fixed to the body (2). Other systems are assembled on these two main components (body and mounted handle) by their functions. Needle slot thrusting handle (3) is movable back and forth in Pivot point for the needle slot thrusting handle (4).

This movement will be transferred to control rod in the needle slot, that is mounted to the other side, and by the means of that rod, to the needle slot (6). Intersection point is the needle slot control rod hole(7)

Needle slot, with back and forth movements of control rod will perfom circular movements on needle slot pivot point (when handle is pulled back to mounted handle needle slot will move forward.)

Control handle for needle capture grip (9) is linked to the handle fixed to the body by a pivot point (10) and performs back-forth movements on that pivot point.

These movements will be transferred to the control handle for needle capture grip (9), located between the pivot point and the other side of the handle, control rod for needle capture grip (11) and needle capture grips (12) control mechanism.

Therefore when this control handle for needle capture grip (9) moves to the mounted handle (2), the control rod for needle capture grip (11) is pulled and the needle capture grips (12) approach each other to catch the needle. Needle capture grips are positioned in such a way to receive the needle in-between after needle comes out of the tissue.

When the rod is pulled (the handle is pressed) grips approach to each other with the aid of the clamp side supports and squeezes the needle. The control of the control rod for needle capture grip is done by the needle slot thrusting handle (3).

When this rod approaches to the body with the help of the finger (so the needle is placed between the grips) the control rod for needle capture grip is pressed during the last 1 cm of the movement. And both these approaches to the handle fixed to the body.

When it is closely approached, a lock mechanism at the end of the the control rod for needle capture starts functioning and fixes needle between the grips. Without pressing second time, this lock mechanism can impossibly be opened.

When the needle capture grips are pulled towards the handle, they are approached each other with the help of the clamp side supports (14) and they squeeze the needle alltogether.

The grips have a spring mechanism from the middle to the outer sides. When they pushed towards, they are seperated and evolving to a position to receive the needle in between.

The needle is in the needle slot (6) which is also in the needle channel (15). Front side of needle channel (17) (back side of the body) is also designed in accordance with pitch of the needle. The front side (17) is mainly composed of the needle clamp (20). Side walls for needle channel (18) is a little bit wider than the needle in order to allow the needle for a free movement when it enters and exits from the channel. The last standing point of the needle from either front to back, or from sides to the bottom is conical shaped (19) and is aimed to adapted to the different sizes of needles.

The needle clamp is under control of the spring (23) with the help of the spring hole (22). Spring pivot point shaft (24) is near to the conical shaped needle channel bottom and nearby the needle body. The place where spring takes its strength is the side of body extension (25) . The spring gets pressed here and pushes the clamp (20) towards the needle. This movement of the needle clamp takes place in the needle clamp slot located in the needle slot (21). The movement of the spring that coincides with the channel will happen in the spring window (26). The top and bottom sides of the needle clamp touching the needle are tilted internally in order to grip the needle (27). The hardness of the spring is so adjusted that it does not allow the needle to move to right or left. The spring, in the normal position, is arranged to push the needle behind the channel (16). The movement, that is away from the needle and allows to release the channel for taking off the needle, of the clamp is provided by needle channel opening wire (28). There are parts in order to take the clamp in from both sides at the end of the wire (29). Clamp connection rod (30) provides the connection between those 2 parts and the clamp. With the help of this rod, those parts in both parts and the clamp are connected. However, clamp connection hole (31) has a flexibility to permit the clamp to make a full circular movement. The parts that supply connection to the rod on both sides of the clamp are connected to the needle channel opening wire (28) rigidly. This wire reaches through the body to the handle and is mounted near to the middle section of the needle channel opener handle (32). This handle would have a movement back and forth over the needle channel opener handle Pivot point (33). Therefore, when release button for the needle channel (34) is pressed towards to the handle fixed to the body, the needle clamp will be pulled away from the needle and the needle is allowed to be placed into the channel. Needle channel opener handle is pushed away from the handle fixed to the body with the help of the spring (35). So that it helps for correct positioning for first usage. Needle channel opener handle is positioned between (2) the handle fixed to the body and the control handle for needle capture grip (9). There is a lock mechanism hole (36) in the handle made from the corrugated sheet, which allows the lock mechanism to work freely (13). The reason why this mechanism is adjusted in such a way is to allow the needle to be passed through the tissue (flesh), and to be captured and to be released with a single stitch movement by the effective working of the grips. When release button for the needle channel (34), located at the end of the needle channel opener handle, is pressed once towards to the handle fixed to the body; needle channel is opened and can be utilized for the needle use.

### Application of the invention:

The device, which is the needle control mechanism mounted upon, is handled by using the handle (2) fixed to the body (1). At the starting position, the lock mechanism of control handle for needle capture grip (13) is not used and it is pushed towards by the needle channel opener handle (32) with the effect of needle channel opener handle spring (35). In this case, needle capture grips (12) are opened and ready to receive the needle in between. Also in this case, the needle clamp (20) is already available. To place the needle in its place, needle slot thrusting handle (3) will be pulled to its midway of the movement. In this position, the control of needle channel from the handle will be much more effective. To place the needle into the channel, release button for the needle channel (34) will be pressed towards to the handle fixed to the body. This movement will reach to the needle clamp (20) through needle channel opening wire (28), clamp control parts (29) and clamp connection rod. Needle clamp will be pulled to the body making the channel for the reception of the needle. The needle will be pushed towards to the channel from its sutured side. When the needle is placed, the button is released. With the help of the strong spring (23) needle clamp is pushed towards the needle and the needle is clamped. The device is ready to use. Depending on the operation in which it is going to be used, it will be taken to the proximity of the suture area through a pipe or directly. Surgeon directs the needle to the tissue by pulling the needle slot thrusting handle (3) towards the handle fixed to the body. When slot thrusting handle (3) approaches to the handle fixed to the body, it pulls the needle channel opener handle (32) with it. At the same time, the grips are approaching to the needle that is already placed in between and the needle clamp is releasing the needle. At the final position, the lock mechanism starts functioning and the needle is fixed between the grips. Thus, the needle passes through the tissue and is caught at the same time. Needle slot thrusting handle is pushed away the handle fixed to the body. So the needle is released from the channel. With the help of the lock mechanism, needle is discarded and stayed as it is. When the needle slot thrusting handle is pushed away totally, the needle is released from the channel (15) and the tissue and be prepared for the second suture. In this stage, needle is fixed in the grips at the end and the lock mechanism at the handle is on, the channel is open. When the needle slot thrusting handle (3) is pulled again, the part of the needle with the suture again enters the channel. At last stage, the needle is place in the channel perfectly. The lock mechanism has such a construction that it stops functioning when it hits to the final point, so the needle capture grips are freed and opened. At the same time, the clamp is also freed from the lock and moves back and forth to squeeze the needle. Device is ready for the new suturing.

## Claims

1. A remote control mechanism for atraumatic surgical needle that has the minimal components for performing remote suturing operation, the mechanism comprising:
a body (1);
a handle (2) fixed to the body (1);
a needle slot thrusting handle (3);
a pivot point (4) for the needle slot thrusting handle (3);
a control rod (5) for the needle slot;
a needle slot (6),
a needle slot control rod hole (7),
a needle slot pivot point (8);
a control handle (9) for a needle capture grip,
a pivot point (10) for the control handle (9) of the needle capture grip;
a control rod (11) for the needle capture grip;
needle capture grips (12);
the lock mechanism (13) of the control handle (9) for the needle capture grip;
clamp side supports (14) for gripping the needle;
a needle channel (15) comprising: a back side (16), a front side (17), side walls (18) and a conical shaped bottom (19);
a needle clamp (20) comprising: a spring hole (22), a clamp connection hole (31) and curved needle clamp sides (27);
a needle clamp slot (21) defined between the needle slot (6) and the needle clamp (20),
a spring (23), a spring pivot shaft (24), a body extension (25), a spring window (26) defined in the needle slot (6);
a needle channel opening wire (28);
clamp control parts (29);
a clamp connection rod (30);
a needle channel opener handle (32);
a needle channel opener handle pivot point (33),;
a release button (34) for the needle channel,
a needle channel opener handle spring (35); and
a hole (36) of lock mechanism;
wherein the pivotal triggering levers (3, 9, 32) for the needle slot thrusting handle (3), the control handle (9) for the needle capture grip (13) and the needle channel opener handle (32) are arranged in such a relation to each other and to the body handle (2) that they are sequentially operable with a same movement of the fingers of the hand holding the device.

2. A remote control mechanism for atraumatic surgical needle as set forth in Claim 1, wherein the needle capture grips (12) approach each other when pulled in order to fix the needle.

3. A remote control mechanism for atraumatic surgical needle as set forth in Claim 1, whereby it is adjustable to any size of needle by taking advantage of its needle channel's conical bottom shape (19), which does not permit the needle to fall off.

4. A remote control mechanism for atraumatic surgical needle as set forth in Claim 1, wherein the mechanism provides a needle clamp (20) that pushes to the bottom of the channel thus, fixing the needle which is already inserted into the needle channel (15), the needle clamp (20) being located in its own needle slot (21) carved just a little bigger than the needle, all together in the needle slot, and having an ability of moving towards or backwards of the needle and to be pushed tightly with the control of spring (23), which is located in the spring hole (22).

5. A remote control mechanism for atraumatic surgical needle as set forth in Claim 1, wherein side of the needle clamp facing towards the needle is straight and pushed bi-directionally to the ends thus, distributing the pressure evenly to these ends, these two ends (27) facing to the needle being curved in accordance with oblique shape of the needle.

6. A remote control mechanism for atraumatic surgical needle as set forth in Claim 1, whereby the processes of pulling the needle clamp and opening the needle channel in allowance of placing the needle are controlled by the needle channel opener handle (32) and whereby applying pressure to this handle towards the body handle (2) also results in withdrawal of the needle clamp away from the needle.

7. A remote control mechanism for atraumatic surgical needle as set forth in any of the preceding Claims 1 - 6, whereby the needle channel opener handle (32) is connected to the body (1) and turnable over the needle channel opener handle pivot point (33) and the needle channel opening wire (28) by virtue of the wire assembly point existing between the pivot point and other end of the handle, and wherein, when pressed towards the body by using the body handle (2), the handle (32) is also able to pull the needle channel opening wire (28) and the clamp control parts (29) in the same direction towards the handle, the parts being mounted to the other side of the wire together with the triggering clamp connection rod (30) and located in the clamp connection hole (31).

8. A remote control mechanism for atraumatic surgical needle as set forth in Claim 7 , whereby the needle clamp (20) is turnable over the point with clamp control parts (29) affecting the clamp connection rod (30) located in the clamp connection hole (31).

9. A remote control mechanism for atraumatic surgical needle as set forth in Claim 1 or 6, whereby a release button (34) is provided for the needle channel being located at the extension of the needle channel opener handle (32) in order to make the needle channel opening easier and by virtue of which, the needle channel is released without touching the other ends.

10. A remote control mechanism for atraumatic surgical needle as set forth in Claim 1 or 4, whereby a strong spring (23) is provided to compress the needle, wherein the spring pushes the needle clamp (20), it gathers its strength from the side of body extension (25) or apropriate point, which is located in the needle slot (6).

11. A remote control mechanism for atraumatic surgical needle as set forth in Claim 1, wherein a two-sided spring window (26) is located in the needle slot (6) allowing back and forth movement of the needle clamp (20) under the pressure of channel opening wire (28) and the spring (23).

12. A remote control mechanism for atraumatic surgical needle as set forth In Claim 1 or 9, wherein the needle channel opener handle (32) is located between the body handle (2) and the control handle (9) for needle capture grip (12) thus allowing the lock mechanism (13) to work Independently with the help of the hole (36) of lock mechanism (13) and at the same time permitting the needle channel to be opened for access of the needle as it moves independently towards the body handle (2).

13. A remote control mechanism for atraumatic surgical needle as set forth in Claim 12, wherein the needle capture grips handle (9) and the needle channel opener handle (32) are being placed respectively between the needle slot thrusting handle (3) and the body handle (2) enabling to handle all controls in accordance with movement of the needle slot thrusting handle (3), whereby, when the needle slot thrusting handle approaches to the mounted handle, it takes other two handles (9, 32) towards the handle fixed to the body (1), so that when the needle passes through just after the flesh, first the needle clamp grips (20) take action and fix the needle rigidly, thus allowing the needle channel to be opened for exit and entrance of the needle into the needle channel through its rear end.

## Patentansprüche

1. Fernsteuerungsmechanismus für eine atraumatische chirurgische Nadel, der zumindest die zur Durchführung eines ferngesteuerten Wundverschlusses notwendigen Komponenten umfasst, wobei folgende Bestandteile zum Mechanismus gehören:
ein Gehäuse (1);
ein Griff (2), der am Gehäuse befestigt ist (1);
Ein Nadelschacht-Schubgriff (3);
ein Drehpunkt (4) für den Nadelschacht-Schubgriff (3);
ein Steuerstab (5) für den Nadelschacht;
ein Nadelschacht (6),
ein Loch im Steuerstab für den Nadelschacht (7),
ein Nadelschacht-Drehpunkt (8);
ein Bedienungsgriff (9) für eine Nadelrückhalteklemme,
ein Drehpunkt (10) für den Bedienungsgriff (9) der Nadelrückhalteklemme;
ein Steuerstab (11) für die Nadelrückhalteklemme;
Nadelrückhalteklemmen (12);
der Sperrmechanismus (13) des Bedienungsgriffs (9) für die Nadelrückhalteklemme;
seitliche Klemmhalterungen (14) zum Festhalten der Nadel;
ein Nadelkanal (15) bestehend aus: einer Rückseite (16), einer Vorderseite (17),
Seitenwänden (18) und einem konisch geformten Ende (19);
eine Nadelklemme (20) bestehend aus: ein Federungsloch (22), ein Klemmverbindungsloch (31) und gebogene, seitliche Nadelklemmen (27);
ein Nadelklemmschacht (21), der sich zwischen dem Nadelschacht (6) und der Nadelklemme (20) befindet,
eine Feder (23), eine Feder-Gelenkachse (24), eine Gehäuse-Verlängerung (25) und ein Federfenster 26) im Nadelschacht (6);
ein Draht zum Öffnen des Nadelkanals (28);
Klemmsteuerungsteile (29);
ein Klemmverbindungsstab (30);
ein Griff zum Öffnen des Nadelkanals (32);
ein Drehpunkt am Griff zum Öffnen des Nadelkanals (33);
Eine Entriegelungstaste (34) für den Nadelkanal,
eine Feder für den Griff zum Öffnen des Nadelkanals (35); und
ein Loch (36) für den Sperrmechanismus;
wobei die scharnierenden Hebel (3, 9, 32) für den Nadelschacht-Schubgriff (3), den Bedienungsgriff (9) für die Nadelrückhalteklemme (13) und den Griff zum Öffnen des Nadelkanals (32) so zueinander und zum Gehäusegriff (2) angeordnet sind, dass sie nacheinander mit einer einzigen Bewegung der Finger an der Hand, die das Gerät festhält, bedient werden können.

2. Fernsteuerungsmechanismus für eine atraumatische chirurgische Nadel nach Anspruch 1, bei dem sich die Nadelrückhalteklemmen (12) beim Anziehen gegeneinander klemmen und damit die Nadel fixieren.

3. Fernsteuerungsmechanismus für eine atraumatische chirurgische Nadel nach Anspruch 1, der dank des konisch geformten Endes des Nadelkanals (19), das ein Herausfallen der Nadel verhindert, auf jede Nadelgröße einstellbar ist.

4. Fernsteuerungsmechanismus für eine atraumatische chirurgische Nadel nach Anspruch 1, bei dem der Mechanismus eine Nadelklemme (20) an die Unterseite des Kanals drückt und dadurch die im Nadelkanal (15) befindliche Nadel an ihrer Stelle hält. Diese Nadelklemme (20) liegt in einem eigenen Nadelschacht (21), der nur geringfügig größer ausgefräst ist als die Nadel selbst, die ebenfalls im Nadelschacht liegt. Dort kann die Nadelklemme vorwärts und rückwärts zur Nadel bewegt werden und mithilfe einer Feder (23), die sich im Federloch (22) befindet, fest angedrückt werden.

5. Fernsteuerungsmechanismus für eine atraumatische chirurgische Nadel nach Anspruch 1, bei dem die Nadelklemme auf der Seite der Nadel gerade ist und in beide Richtungen gegen die Enden drückt; Damit wird der Druck gleichmäßig auf diese Enden verteilt. Diese beiden Enden (27) auf der Seite der Nadel sind ebenso gebogen wie die Nadel selbst.

6. Fernsteuerungsmechanismus für eine atraumatische chirurgische Nadel nach Anspruch 1, bei dem sowohl das Anziehen der Nadelklemme als auch das Öffnen des Nadelkanals zum Einlegen der Nadel, mithilfe des Griffs zum Öffnen des Nadelkanals (32) gesteuert werden, wobei die Druckeinwirkung auf diesen Griff in Richtung des Gehäusegriffs (2) bewirkt, dass die Nadelklemme ihren Griff auf die Nadel löst und sich öffnet.

7. Fernsteuerungsmechanismus für eine atraumatische chirurgische Nadel nach jedem der oben genannten Ansprüche 1-6, bei dem der Griff zum Öffnen des Nadelkanals (32) am Gehäuse (1) befestigt ist und über den Drehpunkt am Griff zum Öffnen des Nadelkanals (33) und mithilfe des Drahts zum Öffnen des Nadelkanals (28) gedreht werden kann. Dies erfolgt an der Drahtspannstelle, die sich zwischen dem Drehpunkt und dem anderen Ende des Griffs befindet. Wird mithilfe des Gehäusegriffs (2) Druck auf das Gehäuse ausgeübt, dann betätigt der Griff (32) gleichzeitig den Draht zum Öffnen des Nadelkanals (28) und die Klemmsteuerungsteile (29) und zieht diese in Richtung des Griffs. Die Teile sind gemeinsam mit dem auslösenden Klemmverbindungsstab (30) auf der anderen Seite des Drahts befestigt und liegen im Klemmverbindungsloch (31).

8. Fernsteuerungsmechanismus für eine atraumatische chirurgische Nadel nach Anspruch 7, bei dem die Nadelklemme (20) an einem Punkt über die Klemmsteuerungsteile (29), die den Klemmverbindungsbolzen (30) im Klemmverbindungsloch (31) ansteuern, gedreht werden kann.

9. Fernsteuerungsmechanismus für eine atraumatische chirurgische Nadel nach Anspruch 1 oder 6, mit einer Entriegelungstaste (34) für den Nadelkanal, die sich an der Verlängerung des Griffs zum Öffnen des Nadelkanals (32) befindet, mit der das Öffnen des Nadelkanals vereinfacht werden soll und mit deren Hilfe der Nadelkanal entriegelt wird, ohne die anderen Enden zu berühren.

10. Fernsteuerungsmechanismus für eine atraumatische chirurgische Nadel nach Anspruch 1 oder 4, mit einer starken Feder (23) zum Zusammendrücken der Nadel, wobei diese Feder, deren Stärke von den Gehäuseverlängerungen (25) oder einem passenden Punkt im Nadelschacht (6) bedingt ist, die Nadelklammer (20) andrückt.

11. Fernsteuerungsmechanismus für eine atraumatische chirurgische Nadel nach Anspruch 1, der auf beiden Seiten des Nadelschachts (6) ein Federfenster (26) aufweist, wobei durch die Spannung des Drahtes zum Öffnen des Nadelkanals (28) und die Feder (23) ein Vor- und Zurückbewegen der Nadelklemme (20) ermöglicht wird.

12. Fernsteuerungsmechanismus für eine atraumatische chirurgische Nadel nach Anspruch 1 oder 9, bei dem sich der Griff zum Öffnen des Nadelkanals (32) zwischen dem Gehäusegriff (2) und dem Steuerungsgriff (9) für die Nadelrückhalteklemme (12) befindet, wodurch der Sperrmechanismus (13) mithilfe der Öffnung (36) des Sperrmechanismus (13) selbstständig funktioniert. Gleichzeitig ermöglicht er ein Öffnen des Nadelkanals für den Zugriff auf die Nadel, indem er sich unabhängig vom Gehäusegriff (2) bewegt.

13. Fernsteuerungsmechanismus für eine atraumatische chirurgische Nadel nach Anspruch 12, bei dem der Griff für die Nadelrückhalteklemmen (9) und der Griff zum Öffnen des Nadelkanals (32) jeweils zwischen dem Nadelschacht-Schubgriff (3) und dem Gehäusegriff (2) angebracht sind und dadurch alle Steuerungsvorgänge entsprechend der Bewegung des Nadelschacht-Schubgriffs (3) ermöglichen. Bei Betätigung des Nadelschacht-Schubgriffs in Richtung des montierten Griffs, aktivieren diese beiden Griffe (9, 32) den Gehäusegriff (1), sodass, wenn die Nadel unmittelbar hinter der dem zu vernähenden Gewebe hindurch verläuft, erst die Nadelrückhalteklemmen (20) eingreifen und die Nadel festhalten, wodurch sich anschließend der Nadelkanal öffnet, und die Nadel freigibt und über das äußerste Ende wieder in den Nadelkanal einlässt.

## Revendications

1. Mécanisme de commande à distance pour aiguille chirurgicale atraumatique possédant les composants minimaux pour la réalisation de sutures à distance, comprenant :
un corps (1) ;
une poignée (2) fixée sur le corps (1) ;
une poignée de poussée sur la rainure de l'aiguille (3);
un axe de pivotement (4) pour la poignée de poussée sur la rainure de l'aiguille (3) ;
une tige de commande (5) pour la rainure de l'aiguille ;
une rainure de l'aiguille (6),
un orifice de tige de commande de rainure de l'aiguille (7),
un axe de pivotement de rainure de l'aiguille (8) ;
une poignée de commande (9) pour un dispositif de blocage,
un axe de pivotement (10) pour la poignée de commande (9) du dispositif de blocage d'aiguille ;
une tige de commande (11) pour le dispositif de blocage d'aiguille ;
dispositifs de blocage d'aiguille (12) ;
le mécanisme de verrouillage (13) de la poignée de commande (9) pour le dispositif de blocage d'aiguille ; supports latéraux de blocage (14) pour maintenir l'aiguille ;
un canal d'aiguille (15) comprenant : une face arrière (16), une face avant (17), des côtés (18) et un fond de forme conique (19) ;
un bloque-aiguille (20) comprenant : un logement de ressort (22), un orifice de liaison de blocage (31) et des faces latérales cintrées de blocage d'aiguille (27) ;
un logement de blocage d'aiguille (21) défini entre le logement d'aiguille (6) et le bloque-aiguille (20),
un ressort (23), un axe de pivotement de ressort (24), une extension de corps (25), une fenêtre à ressort (26) définie dans le logement d'aiguille (6) ;
un fil d'ouverture de canal d'aiguille (28) ;
des pièces de commande de serrage (29) ;
une tige de liaison de blocage (30) ;
une poignée d'ouverture de canal d'aiguille (32) ;
un axe de pivotement de poignée d'ouverture de canal d'aiguille (33) ;
un bouton de déblocage (34) pour le canal d'aiguille,
un ressort de poignée d'ouverture de canal d'aiguille (35) et un orifice (36) pour le mécanisme de verrouillage;
dans lequel les leviers de déclenchement du pivotement (3, 9, 32) pour la poignée de poussée sur la rainure de l'aiguille (3), la poignée de commande (9) pour le dispositif de blocage d'aiguille (13) et la poignée d'ouverture de canal d'aiguille (32) sont disposés les uns par rapport aux autres et par rapport à la poignée du corps (2) de telle manière qu'ils sont manoeuvrables de manière séquentielle d'un même mouvement des doigts de la main qui tient le dispositif.

2. Mécanisme de commande à distance pour aiguille chirurgicale atraumatique selon la spécification 1, dans lequel les dispositifs de blocage d'aiguille (12) se rapprochent lorsqu'ils sont tirés pour maintenir l'aiguille.

3. Mécanisme de commande à distance pour aiguille chirurgicale atraumatique selon la spécification 1, ajustable à n'importe quelle taille d'aiguille grâce à la forme conique du bas de ce canal d'aiguille (19) qui empêche l'aiguille de tomber.

4. Mécanisme de commande à distance pour aiguille chirurgicale atraumatique selon la spécification 1, dans lequel le mécanisme offre un bloque-aiguille (20) qui pousse au fond du canal, maintenant ainsi l'aiguille qui est déjà insérée dans le canal d'aiguille (15), le bloque-aiguille (20) étant situé dans son propre logement d'aiguille (21) d'une taille légèrement supérieure à celle de l'aiguille, tous dans le logement d'aiguille, et capable de faire avancer ou reculer l'aiguille et d'être fortement appuyé par un ressort (23), situé dans le logement de ressort (22).

5. Mécanisme de commande à distance pour aiguille chirurgicale atraumatique selon la spécification 1, dans lequel le côté du bloque-aiguille orienté vers l'aiguille est droit et poussé vers les extrémités, répartissant ainsi uniformément la pression vers ces extrémités, ces deux extrémités (27) orientées vers l'aiguille étant courbées en suivant la forme oblique de l'aiguille

6. Mécanisme de commande à distance pour aiguille chirurgicale atraumatique selon la spécification 1, dans lequel les opérations de traction sur le bloque-aiguille et d'ouverture du canal d'aiguille pour permettre la mise en place de l'aiguille sont commandées par la poignée d'ouverture de canal d'aiguille (32) et dans lequel l'exercice d'une pression sur cette poignée en direction de la poignée du corps (2) amène le bloque-aiguille à s'écarter de l'aiguille

7. Mécanisme de commande à distance pour aiguille chirurgicale atraumatique selon une des spécifications précédentes 1 à 6, dans lequel la poignée d'ouverture de canal d'aiguille (32) est reliée au corps (1) et peut être basculée autour de l'axe de pivotement de la poignée d'ouverture de canal d'aiguille (33) et du fil d'ouverture de canal d'aiguille (28) grâce au point de fixation du fil existant entre l'axe de pivotement et l'autre extrémité de la poignée, et dans lequel, lorsqu'elle est comprimée en direction du corps à l'aide de la poignée du corps (2), la poignée (32) est également capable de tirer sur le fil d'ouverture de canal d'aiguille (28) et les pièces de commande de serrage (29) dans la même direction vers la poignée, les pièces étant montées sur l'autre côté du fil avec la tige de liaison de blocage (30) et situées dans l'alésage de liaison de blocage (31).

8. Mécanisme de commande à distance pour aiguille chirurgicale atraumatique selon la spécification 7, dans lequel le bloque-aiguille (20) peut pivoter, les pièces de commande de blocage (29) agissant sur la tige de liaison de blocage (30) située dans l'alésage de liaison de blocage (31).

9. Mécanisme de commande à distance pour aiguille chirurgicale atraumatique selon la spécification 1 ou 6, dans lequel un bouton de déblocage (34) est prévu pour le canal d'aiguille situé dans le prolongement de la poignée d'ouverture de canal d'aiguille (32) pour faciliter l'ouverture du canal d'aiguille et par l'effet duquel le canal d'aiguille est libéré sans toucher les autres extrémités.

10. Mécanisme de commande à distance pour aiguille chirurgicale atraumatique selon la spécification 1 ou 4, dans lequel un puissant ressort (23) est prévu pour comprimer l'aiguille, où le ressort pousse le bloque-aiguille (20), sa force provient du côté du prolongement du corps (25) ou d'un point qui convient, situé dans le logement d'aiguille (6)

11. Mécanisme de commande à distance pour aiguille chirurgicale atraumatique selon la spécification 1, dans lequel une fenêtre à ressort (26) à deux faces est située dans le logement d'aiguille (6) permettant le mouvement aller et retour du bloque-aiguille (20) sous la pression du fil d'ouverture du canal (28) et du ressort (23).

12. Mécanisme de commande à distance pour aiguille chirurgicale atraumatique selon la spécification 1 ou 9, dans lequel la poignée d'ouverture de canal d'aiguille (32) est située entre la poignée du corps (2) et la poignée de commande (9) du dispositif de blocage d'aiguille (12), permettant ainsi le fonctionnement indépendant du mécanisme de verrouillage (13) avec l'aide de l'alésage (36) du mécanisme de verrouillage (13) qui permet en même temps l'ouverture du canal d'aiguille pour l'accès de l'aiguille qu'il déplace indépendamment vers la poignée du corps (2).

13. Mécanisme de commande à distance pour aiguille chirurgicale atraumatique selon la spécification 12, dans lequel la poignée du dispositif de blocage d'aiguilles (9) et la poignée d'ouverture du canal d'aiguille (32) sont placées respectivement entre la poignée de poussée sur la rainure de l'aiguille (3) et la poignée du corps (2), ce qui permet de traiter toutes les commandes en fonction du mouvement de la poignée de poussée sur la rainure de l'aiguille (3), dans lequel, lorsque la poignée de poussée sur la rainure de l'aiguille approche de la poignée montée, elle amène les deux autres poignées (9, 32) vers la poignée fixée sur le corps (1), de telle sorte que, lorsque l'aiguille la traverse juste après la chair, les mâchoires bloque-aiguille (20) agissent en premier et bloquent l'aiguille de manière rigide, permettant ainsi l'ouverture du canal d'aiguille pour la sortie et l'entrée de l'aiguille dans le canal d'aiguille par son extrémité arrière.
